# DEMANDE DE BREVET EUROPEEN

(11) **EP 0 695 551 A2**
(43) Date de publication de la demande: **07.02.1996**
(21) Numéro de dépôt: 95401818.0
(22) Date de dépôt: 02.08.1995
(51) Int. Cl.: A61K 35/78, A61K 31/01

(54) **Composition laxative contenant un laxatif de lest et un laxatif lubrifiant**

(30) Priorité: 04.08.1994 FR 9409697
(71) Demandeur: Chicouri, Marcel, F-75006 Paris (FR)
(72) Inventeur: Chicouri, Isabelle, F-75006 Paris (FR); Chicouri, Marcel, F-75006 Paris (FR)
(74) Mandataire: Burtin, Jean-François

(57) **Abrégé**

La présente invention se rapporte à de nouvelles compositions pharmaceutiques à action laxative.

Elle se rapporte plus précisément à des compositions pharmaceutiques formées d'un laxatif de lest à caractère mucilagineux et d'un laxatif lubrifiant caractérisées en ce que le laxatif de lest est une poudre de psyllium additionnée de gomme acacia et d'acide phosphorique.

Médicament contre la constipation.

## Description

La présente invention se rapporte à de nouvelles compositions pharmaceutiques à action laxative.

Elle concerne plus particulièrement une composition pharmaceutique renfermant, à titre de principes actifs, un laxatif de lest à caractère mucilagineux et un laxatif lubrifiant.

On connaissait déjà par la demande de brevet français 87.14281 au nom du demandeur, une préparation laxative du même genre où le laxatif lubrifiant était constitué d'huile de paraffine micro-encapsulée dans de la gélatine et éventuellement durcie par tannage pour obtenir une préparation pulvérulente.

Ces deux constituants tout en fournissant une préparation pharmaceutique efficace, présentaient cependant l'inconvénient de ne pas être suffisamment stables et de se détériorer au stockage. Dans ces conditions, notamment par élévation de la température dans les locaux où le médicament était conservé, de l'huile suintait rendant le médicament d'ingestion peu agréable et entraînant des éliminations d'huile intempestives par voie anale.

De la même façon, la demande de brevet français 87.10370 et le brevet européen correspondant 0.301.943, ont décrit une préparation pharmaceutique laxative contenant une paraffine liquide micro-encapsulée dans de la gélatine et de la poudre de psyllium.

Cette demande de brevet ou ce brevet indiquent simplement que la paraffine liquide micro-encapsulée dans la gélatine se présente sous une forme originale sèche. Il n'apparaît pas davantage que la paraffine liquide, sous forme micro-encapsulée dans de la gélatine, garantisse davantage la stabilité de la préparation.

Il se trouve, en outre, que la densité et les caractéristiques mécaniques des deux constituants sont fondalement différentes et, il en résulte une grande difficulté à obtenir un mélange homogène de poudres où les quantités de principes actifs peuvent être définies d'une manière exacte.

Il se trouve enfin, que la demande de brevet français 87.10370 indique que le produit pharmaceutique laxatif est destiné à être dispersé dans un grand verre d'eau ou de jus de fruit avant l'ingestion. On voit donc à quel point la stabilité des micro-capsules joue un rôle fondamental car la gélatine de ces micro-capsules se dissout plus ou moins dans un véhicule aqueux et laisse passer, à ce moment, de la paraffine liquide.

Ces inconvénients avaient déjà trouvé une solution beaucoup plus satisfaisante en réalisant des micro-granules de paraffine dans de la gélatine puis en durcissant la paroi de ces micro-capsules et en la rendant imperméable par réaction chimique (cross linking) avec de l'adehyde glutarique. On sait, en effet, que l'adehyde glutarique est un dialdehyde apte à réagir avec des fonctions aminées en formant des bases de Schiff très peu solubles dans l'eau et peu susceptibles d'être attaquées ou digérées par des sucs digestifs en milieu acide, en raison de la nature polymérique linéaire ou pontée des enchaînements ainsi formés. Les combinaisons formées avec la gélatine sont ainsi beaucoup plus résistantes mécaniquement, chimiquement et physiquement. Les micro-granules ainsi durcis sont difficilement altérables et leur conservation dans toutes les conditions d'hygrométrie et de température, peut être raisonnablement garantie sur une longue période.

Ces micro-granules ne peuvent être dégradés qu'en milieu alcalin comme, par exemple, dans l'intestin grêle.

En outre, pour améliorer la fluidité et la miscibilité de ces micro-granules à la poudre de téguments mucilagineux, il était apparu convenable de les additionner d'un excipient inerte très finement pulvérulent. Un tel excipient est de préférence une silice colloïdale comme l'Aerosil 100 ou l'Aerosil 200 ou bien une terre siliceuse purifiée comme le Syloid 244. La teneur en paraffine liquide dans de tels micro-capsules est comprise entre 82 et 92% de la masse totale et de préférence entre 84 et 90%.

Pour des raisons semblables, il était apparu avantageux d'incorporer à la poudre de Psyllium, une alcoyl cellulose susceptible de contribuer à améliorer les propriétés physiques de la poudre de psyllium.

Or, les demandeurs ont trouvé maintenant qu'une solution sensiblement plus simple au problème de l'homogénéité des poudres et à la bonne conservation du mélange ainsi obtenu, pouvait être trouvé en utilisant une qualité particulière de poudre de Psyllium ayant une granulométrie et une fluidité parfaitement adaptée au mélange avec la préparation de micro-capsules d'huile de paraffine, une meilleure dispersibilité dans l'eau, et par conséquent, la formation d'un gel à propriétés mécaniques améliorées, ainsi qu'en outre, des propriétés organoleptiques améliorées.

Cette qualité de poudre de Psyllium est obtenue par broyage et vannage de la poudre de semences de Plantago ovata.

La poudre ainsi obtenue après nettoyage, est broyée à nouveau 6 ou 7 fois pour l'élimination totale des enveloppes. Le produit broyé est tamisé pour recueillir la fraction ayant la finesse désirée. Finalement, la poudre est additionnée de 10% de gomme acacia et d'acide phosphorique. La gomme acacia joue un rôle d'agent liant. L'acide phosphorique sert d'agent tampon et est présent en parties par million, sa présence contribue à donner au mucilage un pH très légèrement acide, ce qui est favorable pour le gonflement. Ce pH est voisin de 4.

Le psyllium broyé ainsi traité et additionné, présente une grande fluidité qui permet son incorporation aux micro-granules d'huile de paraffine liquide sans risque de démixtion et surtout avec l'assurance que le mélange sera parfaitement homogène et se conservera parfaitement.

Les proportions des deux principes actifs sont notamment de 1 p. d'huile de paraffine pour 2 p. de Psyllium broyé et traité à 2 p. d'huile de paraffine pour 1 p. de Psyllium broyé et traité, avec une préférence pour un rapport à parties égales.

Une formule préférée est celle qui renferme 3,33 g de poudre de Psyllium traitée à l'acide phosphorique et additionnée de gomme acacia et 3,33 g d'huile de paraffine micro-encapsulée, durcie par de l'aldéhyde glutarique et fluidifiée par adjonction d'un excipient inerte très finement pulvérulent comme par exemple, la silice colloïdale, le phosphate tricalcique ou encore le stéarate de calcium.

Le terme "gomme acacia", utilisé ici, est l'équivalent du terme "gomme arabique" employé dans le passé, tout en présentant une signification plus large en n'étant pas limité à l'Acacia vera.

Les compositions pharmaceutiques selon l'invention contiennent en outre un agent liant ou un agent de fluidité.

Les compositions pharmaceutiques selon l'invention peuvent, en outre, être édulcorées par du Xylitol, de l'Aspartame et/ou de la saccharine ou aromatisées comme par exemple, par un arôme d'agrumes ou par un arôme caramel.

Les compositions pharmaceutiques selon l'invention peuvent, en outre, être additionnées d'un agent de conservation.

Les compositions pharmaceutiques selon l'invention se présentent sous forme de préparations pulvérulentes réparties en sachets, en flacons ou en paquets.

Elles sont administrées pour le traitement de la constipation à raison d'une ou deux administrations journalières.

Les exemples suivants illustrent l'invention, ils ne la limitent en aucune façon :

### EXEMPLE I

**Sachets à 6,6 g de mélange**

La poudre de semences de Plantago Ovatamare ou Plantain rose, est nettoyée des poussières et impuretés, puis on sépare les téguments par broyage et vannage. Le broyage est effectué au broyeur avec des billes en émeri ou au moulin équipé de pierres plates. Les semences, après complet nettoyage, sont repassées successivement au broyeur 6 ou 7 fois, pour l'élimination complète des parties ligneuses. Le produit broyé contenant l'épisperme et la partie centrale de la semence est tamisée pour éliminer les amandes après quoi, on la fait passer sur des tamis allant de 30 à 100 mailles de manière à recueillir des poudres de différentes finesses, les plus riches en élément mucilagineux.
La densité apparente de la poudre utilisée est de 2,2 ml/g.
La poudre obtenue est alors mélangée avec 10% de gomme acacia qui agit en tant qu'agent liant, un agent de conservation, s'il y a lieu, et de quelques parties par million d'acide phosphorique. Le mélange est ensuite homogénéisé, séché et tamisé à nouveau.
25 kg de la poudre de Plantago ovatamare, ainsi préparée et traitée, sont ensuite mélangés avec 28 kg de microsphères d'huile de paraffine préalablement durcies par tannage, jusqu'à obtention d'un mélange fluide et homogène.

La poudre est ensuite tamisée sur un tamis 200 et le mélange très fluide est édulcoré avec 0,100 g de saccharinate de calcium et 15 g d'arôme orange adsorbé sur de la silice colloïdale.
Le mélange est ensuite réparti en sachets de 6,6 g renfermant un mélange en proportions égales de poudre de Plantago Ovatamare traité et d'huile de paraffine microencapsulée.

### EXEMPLE II

**Sachets à 10 g de mélange**

On mélange 400 g de poudre de Plantago ovatamare préalablement traitée comme ci-dessus avec 600 g de microsphères d'huile de paraffine jusqu'à parfaite homogénéité. On ajoute au mélange 60 g de phosphate tricalcique et 40 g de silice colloïdale (Aerosil 200). Le mélange est réparti en sachets de 10 g.
Le contenu d'un sachet est destiné à être versé dans un 1/2 verre d'eau. Le lait résultant est aisément ingérable.

## Revendications

1. Nouvelles compositions laxatives à base d'un laxatif de lest à caractère mucilagineux et un laxatif lubrifiant caractérisées en ce qu'elles renferment, à titre de laxatif de lest, une poudre de Psyllium ovata additionnée de gomme acacia et d'acide phosphorique.

2. Nouvelles compositions laxatives selon la revendication 1° dans lesquelles la poudre de Psyllium est obtenue par broyage et tamisage répétés pour obtenir une séparation complète des enveloppes et l'élimination des fragments d'amande.

3. Nouvelles compositions laxatives selon la revendication 1° ou la revendication 2° dans lesquelles on additionne la poudre de Psyllium de 10% environ de gomme acacia.

4. Nouvelles compositions pharmaceutiques selon la revendication 1° ou la revendication 2° dans lesquelles la poudre de Psyllium est additionnée d'acide phosphorique de manière à ce que le mucilage dans l'eau présente un pH de l'ordre de 4.

5. Nouvelles compositions pharmaceutiques selon l'une des revendications 1 à 4° dans lesquelles le laxatif lubrifiant se présente sous forme d'huile de paraffine micro-encapsulée, durcie par de l'adéhyde glutarique et fluidifiée.

6. Nouvelles compositions pharmaceutiques selon l'une des revendications 1 à 5° dans lesquelles les proportions de poudre de Psyllium broyée et traitée à l'huile de paraffine micro-encapsulée varie de 1 partie d'huile de paraffine pour 2 parties de Psyllium à 2 parties d'huile de paraffine pour 1 partie de Psyllium.

7. Une composition pharmaceutique selon l'une des revendications 1 à 6° dans laquelle l'huile de paraffine micro-encapsulée et la poudre de Psyllium broyée et traitée sont à parties égales.

8. Une composition pharmaceutique selon l'une des revendications 1 à 7° dans laquelle les principes actifs sont dilués avec un agent liant ou un agent de fluidité.
